(19) Europäisches Patentamt
European Patent Office
Office européen des brevets

(11) Publication number: **0 488 348 A1**

# (12) EUROPEAN PATENT APPLICATION

(21) Application number: **91120483.2**

(22) Date of filing: **29.11.91**

(30) Priority: **30.11.90 JP 329646/90**

(43) Date of publication of application:
**03.06.92 Bulletin 92/23**

(84) Designated Contracting States:
**BE DE FR GB NL**

(51) Int. Cl.[5]: **C07D 249/08**, A01N 43/653

(71) Applicant: **KURKEHA KAGAKU KOGYO KABUSHIKI KAISHA**
**1-9-11, Nihonbashi, Horidome-cho**
**Chuo-ku Tokyo 103(JP)**

(72) Inventor: **Saishoji, Toshihide**
**154-1, Harada, Nishiki-machi**
**Iwaki-shi, Fukushima-ken 974(JP)**
Inventor: **Ito, Atsushi**
**55-1, Sekishita, Nishiki-machi**
**Iwaki-shi, Fukushima-ken 974(JP)**
Inventor: **Kumazawa, Satoru**
**61-14, Minamidai, Kanayama-machi**
**Iwaki-shi, Fukushima-ken 974(JP)**

(74) Representative: **Kraus, Walter, Dr. et al**
**Patentanwälte Kraus, Weisert & Partner**
**Thomas-Wimmer-Ring 15**
**W-8000 München 22(DE)**

## (54) Optically active triazole derivatives and fungicide compositions.

(57) Disclosed herein is an optically active (-)-triazole derivative represented by the following general formula (I), which has a configuration that R [1] and the nonsubstituted or substituted phenylmethyl group are bonded to the cis-position of the hydroxyl group and $R^2$ is bondend to the trans-position of the hydroxyl group:

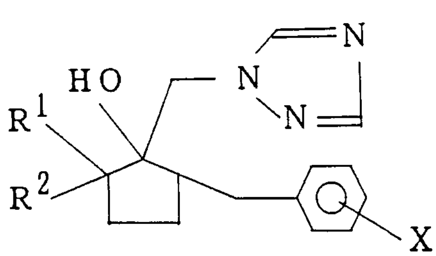

(I)

wherein one of $R^1$ and $R^2$ is a $C_2$ - $C_4$ straight-chain alkyl group and the other is a hydrogen atom, and X denotes a hydrogen atom or a halogen atom.

An agricultural and horticultural fungicide composition comprising the triazole derivative represented by the above-mentioned general formula (I) is also disclosed.

EP 0 488 348 A1

## BACKGROUND OF THE INVENTION

1) Field of the invention

The present invention relates to an optically active capable of effectively utilizing as agricultural and horticultural fungicides and to an agricultural and horticultural fungicide containing it as an active ingredient.

2) Description of the Related Arts:

Japanese patent application laid-open 93574/1989 (EP-A-267778) iscloses a racemic azole derivative represented by the general formula (A) and a process for preparing the same

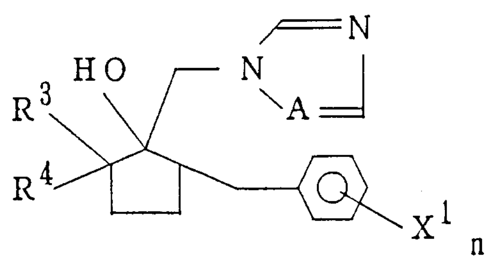

(A)

wherein R $_3$ and $R_4$ denote each a $C_1$ -$C_5$ alkyl group or a hydrogen atom, but $R_3$ and $R_4$ are not the hydrogen atom at the same time, X $^1$ denotes a halogen atom, a $C_1$ -$C_5$ alkyl group or a phenyl group, n is 0 or an integer of 1 or 2, and A denotes a nitrogen atom or CH, and discloses that such derivatives have excellent fungicidal activity and plant growth regulating activity.

By the way, there have been strong demands for agricultural and horticultural fungicides capable of exhibiting reliable activity even at such low application dosages as bringing about the advantage of reducing the amount present in the environment, and agricultural and horticultural fungicides being low in toxicity to animals and plants. It has been hence desired to develop such agricultural and horticultural fungicides.

The present invention has been completed with a view towards meeting such demands.

Accordingly, an object of the present invention is to provide triazole derivatives having excellent fungicidal activities and to provide agricultural and horticultural fungicides containing such derivatives.

## SUMMARY OF THE INVENTION

The present inventors have done earnest studies in order to solve the above mentioned problems. As a result of studies about activities of optical isomers obtained by optical resolution of racemic azole derivatives represented by the formula (A), it has been found that the optical isomers of the present invention have a higher fungicidal activity than the racemic modification therof and that they do not have an activity of inhibiting plant growth which sometimes appears as crop injury, leading to completion of this invention.

The characteristics of the present invention are as follows.

In one aspect of this invention, there is thus provided an optically active (-)-triazole derivative represented by the following general formula (I), which has a configuration that $R^1$ and the nonsubstituted or substituted phenylmethyl group are bonded to the cis position of the hydroxyl group and $R^2$ is bonded to the trans position of the hydroxyl group,

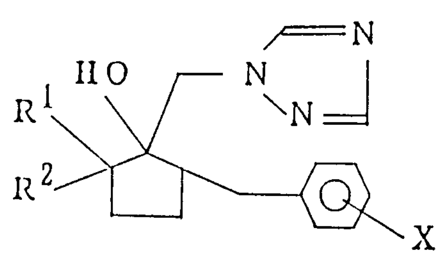

(I)

wherein one of $R^1$ and $R^2$ is a $C_2$ - $C_4$ straight-chain alkyl group and the other is a hydrogen atom, and X denotes a hydrogen atom or a halogen atom.

In another aspect of this invetion, there is also provided an agricultural and horticultural fungicide composition comprising as an active ingredient an optically active (-)-triazole derivative represented by the above mentioned general formula (I) together with an inert carrier or other adjuvants.

## DETAILED DESCRIPTION OF PREFERRED EMBODIMENTS

In the present invention, the above mentioned triazole derivatives may be used as salts of inorganic acids, salts of organic salts or complex salts of metal ions. These salts can be obtained by the prior well-known methods.

According to the present invention, optically active triazole derivative shown in the general formula (I) can be obtained by reacting oxaspiroheptane derivative represented by the general formula (II) with a tirazole represented by the general formula (III) in the following reaction formula described in EP-A-267778 and moreover optical resolution.

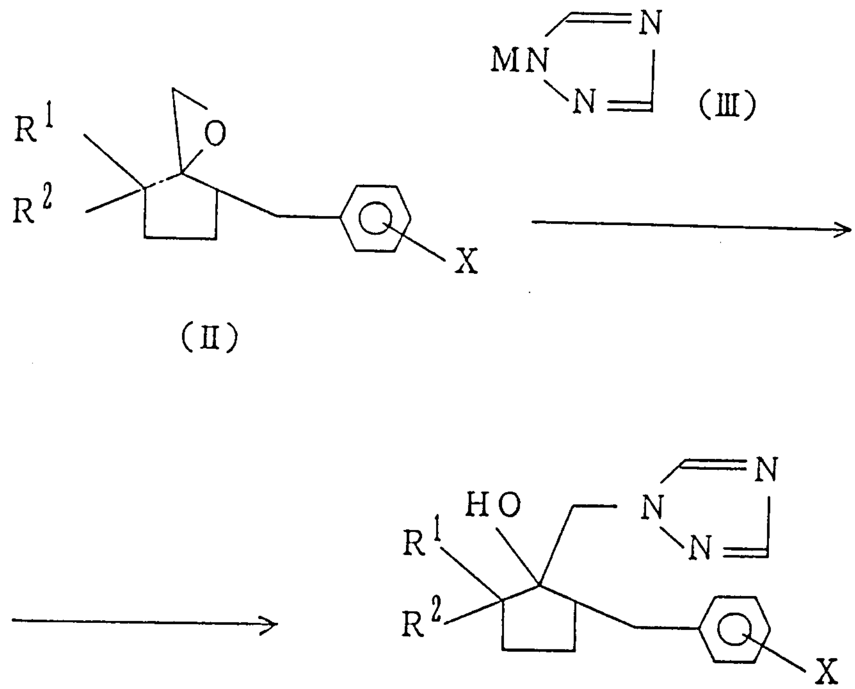

(III)

(II)

(I)

wherein $R^1$ and $R^2$ are the same meaning as defined above, and M denotes a hydrogen atom or an alkali metal.

As for the configuration of the triazole derivative represented by the general formula (I) and compound represented by the general formula (II) in the above described reaction formula, $R^1$ and the substituted or nonsubstituted phenylmethyl group are bonded to the cis position and $R^2$ is bonded to the trans position to the oxygen atom attached to the cyclopentane ring.

For the optical resolution, the triazole derivative represented by the general formula (I) is dissolved in a solvent (for example, metanol), and high performance liquid chromatography with chiral column available on the market (for example, Chiralcell-OD produced by Daisel Chemical Co. Ltd.) is developed with adequately mixed hexane and isopropanol, and optical isomer can be collected fractionally. The solvent is removed from the resultant solution under a reduced pressure, and the compound obtained as a solid is recrystallized from ethyl acetate and hexane, whereby the objective optically active triazole derivative shown in the general formula (I) (hereinafter referred to as "present compound") is obtained.

Specific examples and physical and chemical properties (melting point and specific rotation) of the present compounds are shown in Table 1.

Still more as for its configuration, the present compounds shown in Table 1 include $R^1$ and the non-

substituted or substituted phenylmethyl group in the cis position of the hydroxyl group and $R^2$ in the trans position of the hydroxyl group.

Table 1

| Compound number | $R^1$ | $R^2$ | X | Melting point (°C) | Specific rotation | |
|---|---|---|---|---|---|---|
| | | | | | $[\alpha]_D^{20}$ | c(g/ml) 1) |
| I-1 | $C_2H_5$ | H | 4-Cl | 115-116 | -10.2 | 1.41 |
| I-2 | H | $C_2H_5$ | 4-Cl | 102-103 | -24.4 | 1.11 |
| I-3 | n-$C_3H_7$ | H | 4-Cl | Oil | -16.7 | 1.02 |
| I-4 | n-$C_4H_9$ | H | 4-Cl | Oil | -18.8 | 1.01 |

1) Ethyl alcohol is used as a solvent

The present compounds have high activities as fungicides as compared with racemic modification, and they do not exhibit any activity of inhibiting plant growth which sometimes appears as crop injury. Acccordingly, they exhibit the same effect in a low application dosage as the racemic modification, and they are able to use for a wider range of crop diseases and to use by such a method that crop injury is often induced. Accordingly, they are safe if a high concentration is used by mistake.

Since the present compounds have fungicidal activities on the following wide range of plant diseases, they can be used as fungicides for such plant diseases.

The present compounds have activities to, for example, Pyricularia oryzae on rice plant, Cochliobolus miyabeanus on rice plant, Rhizoctonia solani on rice plant, Helminthosporium sigmoideum on rice plant, Gibberella fujikuroi on rice plant, Podosphaera leucotricha on apple, Venturia inaequalis on apple, Monilinia mali on apple, Alternaria mali on apple, Valsa mali on apple, Alternaria kikuchiana on pear, Phyllactinia pyri on pear, Gymnosporangium asiaticum on pear, Venturia nashicola on pear, Uncinula necator on grape, Phakopsora ampelopsidis on grape, Glomerella cingulata on grape, Erysiphe graminis f.sp.hordei on barley, Rhynchosporium secalis f.sp.hordei on barley, Puccinia graminis on barley, Puccinia striiformis on barley, Puccinia recondita on wheat, Septria tritici on wheat, Puccinia striiformis on wheat, Erysiphe graminis f.sp. tritici on wheat, Sphaerotheca fuliginea on melons, Colletotrichum lagenarium on melons, Fusarium oxysporum f.sp. niveum on watermelon, Fusarium oxysporum f.sp. cucumerinum on cucumber, Fusarium oxysporum f.sp. raphani on japanese radish, Erysiphe cichoracerum on tomato, Alternaria solani on tomato, Erysiphe cichoracearum on egg plant, Sphaerotheca humuli on strawberry, Erysiphe cichoracearum on tobacco, Alternaria longipes on tobacco, Cercospora beticola on sugar beet, Alternaria solani on potato, Septoria glycines on soybean, Cercospora kikuchi on soybean, Monilinia fructicola on stone fruit trees, Botrytis cinerea and Sclerotinia sclerotiorum on various crops, etc.

The present compounds exhibit not only the preventive control effect but also the curative control effect on several diseases among the above.

Though the preset compound can be used alone, they generally be used in the form of dust, wettable powder, granules or emulsion, etc. together with auxiliary agents for preparations. In such a case, the preparations are prepared so as to contain one or more of the present compounds in an amount of 0.1 - 95 % by weight, preferably, 0.5 - 90 % by weight, and more preferably 2 - 70% by weight.

Examples of carriers, diluents and surfactants used as the auxiliary agents for preparations include the following.

Examples of solid carriers include talc, kaolin, bentonite, diatom earth, white carbon and clay, etc.

Examples of liquid diluents include water, xylene, toluene, chlorobenzene, cyclohexane, cyclohexanone, dimethylsulfoxide, dimethylformamide and alcohol, etc.

Examples of the surfactants include polyoxyethylene alkylaryl ether and polyoxyethylene sorbitan monolaurylate etc., as emulsifiers, salts of lignin sulfonic acid, salts of dibutylnaphthalenesulfonic acid, etc., as dispersing agents, and salts of alkylsulfonic acid and salts of alkylphenylsulfonic acid, etc., as wetting agents.

The above preparations are classified into those which can be used directly, and those which are used after diluting so as to have a suitable concentration with a diluent such as water, etc.

The concentration of the present compounds in case of using after diluting is preferred to be in a range

of 0.001 - 1.0%.

Further, the application dosage of the present compounds is in a range of 20 - 5000 g and preferably 50 -1000 g per 1 ha of agricultural and horticultural land such as farm, paddy field, fruit garden, hothouse, etc.

It is of course possible to increase and decrease the concentration and the application dosage beyond the above-mentioned ranges, because they depend upon the form of preparations, method of application, place to be used, target crops, etc.

Furthermore, the present compounds can be used in combination with other effective ingredients, such as fungicides, insectcides, miticides, herbicides, etc.

## EXAMPLES

Preparation examples, formulation examples and test examples are described in the following, by which the present invention is illustrated in detail.

This invention however is not limited to these preparation examples, formulation examples and test examples.

### Example 1

Separation of optically active (-)-(1α ,2α ,5α )-2-[(4-chlorophenyl)methyl]-5-ethyl-1-(1H-1,2,4-triazol-1-ylmethyl)cyclopentanol [Compound(I - 1)]

a) Production of racemic (1 α ,2α ,5α )-2-[(4-chlorophenyl)methyl]-5-ethyl-1-(1H-1,2,4-triazol-1-ylmethyl)-cyclopentanol [$R^1$ = $C_2H_5$ , $R^2$ = H and X = 4-Cl inthe formula (I)]

Into 10 ml of anhydrous dimethylformamide, 630 mg of sodium hydride (prepared by washing 60 % oily sodium hydride with anhydrous benzene) were added and then 1.8 g of 1H-1,2,4-triazole was added, followed by stirring at a room temperarure until bubbling was over. A solution of 3.3 g of racemic ( 3 α ,4α ,7α )-4-[(4-chlorophenyl)methyl]-7-ethyl-1-oxaspiro[2.4]heptane in 6.2 ml of anhydrous dimethylformamide was dropped into the thus obtained solution, and the whole mixture was stirred for 1 hours at 80 ° C.

After leaving the thus obtained reaction mixture to cool, it was poured into iced water, and the mixture wax extracted with methylene chloride to obtain an organic layer. After washing the organic layer with saline water and drying the layer on anhydrous sodium sulfate, the solvent was distilled off from the layer under a reduced pressure. The resultant residue was purified by silica gel column chromatography and was further recrystallized from a mixture of n-hexane and ethyl acetate to obtain 2.96 g of the objective compound.

Yield: 70.3 %.
Melting point: 82 - 84 ° C
NMR: ($CDCl_3$ ); δ ppm
0.67 - 2.23(m,11H), 2.43(d,2H,J = 7),2.93(s,2H),
4.20(s,2H), 0.67 - 2.23(m,4H), 7.93(s,1H,),
8.07(s,1H)

b) Separation of Compound (I - 1)

The racemic triazole derivative prepared in a) was dissolved in methanol and the objective compound was collected fractionally by high performance liquid chromatography with chiral column according to the below-mentioned condition.

The solvent was removed from the obtained solution under a reduced pressure and the residue was recrystallized from ethyl acetate and hexane to obtain optically active (-) - (1α ,2α ,5α )-2-[(4-chlorophenyl)-methyl]-5-ethyl-1-(1H-1,2,4-triazol-1-ylmethyl)cyclopentanol [Compound(I - 1)].

High performance liquid chromatography condition:

| | |
|---|---|
| Column: | Chiralcell-OD, 10mmx L500mm; produced by Daisel Chemical Co. Ltd. |
| Pump: | HITACHI 638 -30; produced by Hitachi Ltd. |
| Eluent: | n-Hexane / ispopropanol mixture = 18.75 / 1 |
| Flow rate: | 10 ml/min |
| Detector: | UV 268nm |
| Injection volume: | 15 mg/150μ l (methanol) |

| | |
|---|---|
| Retention time of compound (I - 1): | 75 minutes |

Example 2

Separation of optically active (-)-(1α ,2α ,5β )-2-[(4-chlorophenyl)methyl]-5-ethyl-1-(1H-1,2,4-triazol-1-ylmethyl)cyclopentanol [Compound(I - 2)]

a) Production of racemic ( 1 α ,2α ,5β )-2-[(4-chlorophenyl)methyl]-5-ethyl-1-(1H-1,2,4-triazol-1-ylmethyl)-cyclopentanol [$R^1$ = H, $R^2$ = $C_2H_5$ and X = 4-Cl in the formula (I)]

Under the same condition described in a) of Example 1, racemic (3 α ,4α ,7β )-4-[(4-chlorophenyl)-methyl]-7-ethyl-1-oxaspiro[2.4]heptane gave racemic (1α ,2α ,5β )-2-[(4-chlorophenyl)methyl]-5-ethyl-1-(1H-1,2,4-triazol-1-ylmethyl)cyclopentanol
Melting point: 93 - 95 °C
NMR: ($CDCl_3$ ); δ ppm
0.70 - 2.13(m,11H), 2.13 - 2.47(m,2H), 3.83(s,1H),
4.00(d,1H,J = 14), 4.30(d,1H,J = 14), 6.88(d,2H,J = 8),
7.18(d,2H,J = 8), 7.93(s,1H), 8.17(s,1H)

b) Separation of Compound (I - 2)

The racemic triazole derivative prepared in a) was dissolved in methanol and the objective compound was collected fractionally by high performance liquid chromatography with chiral column according to the below-mentioned condition.

The solvent was removed from the obtained solution under a reduced pressure and the residue was recrystallized from ethyl acetate and hexane to obtain optically active (-)-( 1 α ,2α ,5β )-2-[(4-chlorophenyl)-methyl]-5-ethyl-1-(1H-1,2,4-triazol-1-ylmethyl)cyclopentanol [Compound(I - 2)]
High performance liquid chromatography condition:

| | |
|---|---|
| Column: | Chiralcell-OD, ∅10mm x L500mm; produced by Daisel Chemical Co. Ltd. |
| Pump: | HITACHI 638 -30; produced by Hitachi Ltd. |
| Eluent: | n-Hexane / ispopropanol mixture = 15 / 1 |
| Flow rate: | 10 ml/min |
| Detector: | UV 268nm |
| Injection volume: | 15 mg/150μ l (methanol) |
| Retention time of compound (I - 2): | 64 minutes |

Example 3

Separation of optically active (-)-(1α ,2α ,5α )-2-[(4-chlorophenyl)methyl]-5-propyl-1-(1H-1,2,4-triazol- 1-ylmethyl)cyclopentanol [Compound(I - 3)]

a) Production of racemic ( 1 α ,2α ,5α )-2-[(4-chlorophenyl)methyl]-5-propyl-1-(1H-1,2,4-triazol-1-ylmethyl)-cyclopentanol [ $R^1$ = n-$C_3H_7$ , $R^2$ = H and X = 4-Cl in the formula (I)]

Under the same condition described in a) of Example 1,except recrystallization, racemic (3α ,4α ,7α )-4-[(4-chlorophenyl)methyl]-7-propyl-1-oxaspiro[2.4]heptane gave racemic (1α ,2α ,5α )-2-[(4-chlorophenyl)-methyl]-5-propyl-1-(1H-1,2,4-triazol-1-ylmethyl)cyclopentanol
Melting point: 83 - 85 °C
NMR: ($CDCl_3$ ); δ ppm
0.61~2.26(m,13H), 2.26 - 2.57(m,2H),
2.51~2.81(b,1H), 4.21(s,2H), 7.03(d,2H, J = 9),
7.23(d,2H,J = 9), 7.96(s,1H), 8.07(s,1H)

b) Separation of Compound (I - 3)

The racemic triazole derivative prepared in a) was dissolved in methanol and the objective compound was collected fractionally by high performance liquid chromatography with chiral column according to the

below-mentioned condition.

The solvent was removed from the obtained solution under a reduced pressure to obtain the optically active (-)-( 1 α ,2α ,5α )-2-[(4-chlorophenyl)methyl]-5-propyl-1-(1H-1,2,4-triazol-1-ylmethyl)cyclopentanol [Compound(I - 3)]

High performance liquid chromatography condition:

| | |
|---|---|
| Column: | Chiralcell-OD, 10mm x L500mm; produced by Daisel Chemical Co. Ltd. |
| Pump: | HITACHI 638 -30; produced by Hitachi Ltd. |
| Eluent: | n-Hexane / ispopropanol mixture =20 / 1 |
| Flow rate: | 10 ml/min |
| Detector: | UV 268nm |
| Injection volume: | 15 mg/150μ l (methanol) |
| Retention time of compound (I - 3): | 74 minutes |

Example 4

Separation of optically active (-)-(1α ,2α ,5α )-2-butyl-5-[(4-chlorophenyl)methyl]-1-(1H-1,2,4-triazol-1-ylmethyl)cyclopentanol [Compound(I - 4)]

a) Production of racemic ( 1 α ,2α ,5α )-2-butyl-5-[(4-chlorophenyl)methyl]-1-(1H-1,2,4-triazol-1-ylmethyl)-cyclopentanol [ $R^1$ = n-$C_4H_9$ , $R^2$ = H, X = 4-Cl in the formula (I)]

Under the same condition described in a) of Example 1, except recrystallization, racemic (3 α ,4α ,7α )-4-butyl-7-[(4-chlorophenyl)methyl]-1-oxaspiro[2.4]heptane gave racemic (1α ,2α ,5α )-2-butyl-5-[(4-chlorophenyl)methyl]-1-(1H-1,2,4-triazol-1-ylmethyl)cyclopentanol
Oily product
NMR: ($CDCl_3$ ); δ ppm
0.60~2.63(m,17H), 2.80(s,1H),4.23(s,2H),
7.07(d,2H,J = 8), 7.27(d,2H,J = 8), 8.00(s,1H),
8.13(s,1H)

b) Separation of Compound (I - 4)

The racemic triazole derivative prepared in a) was dissolved in methanol and the objective compound was collected fractionally by high performance liquid chromatography with chiral column according to the below-mentioned condition.

The solvent was removed from the obtained solution under a reduced pressure to obtain the optically active (-)-( 1 α ,2α ,5α )-2-butyl-5-[(4-chlorophenyl)methyl]-1-(1H-1,2,4-triazol-1-ylmethyl)-cyclopentanol [Compound(I - 4)]

High performance liquid chromatography condition:

| | |
|---|---|
| Column: | Chiralcell-OD, 10mm x L500mm; produced by Daisel Chemical Co. Ltd. |
| Pump: | HITACHI 638 -30; produced by Hitachi Ltd. |
| Eluent: | n-Hexane / ispopropanol mixture =20 / 1 |
| Flow rate: | 10 ml/min |
| Detector: | UV 268nm |
| Injection volume: | 15 mg/150μ l (methanol) |
| Retention time of compound (I - 4): | 76 minutes |

Example 5

Dust:

| | |
|---|---|
| Compound (I - 1) | 3 parts by weight |
| Clay | 40 parts by weight |
| Talc | 57 parts by weight |

The above-mentioned components were pulverized and mixed to prepare a composition of dust form.

Example 6

Wettable powder:

| | |
|---|---|
| Compound (I - 2) | 50 parts by weight |
| Lignin sulfonate | 5 parts by weight |
| Alkyl sulfonate | 3 parts by weight |
| Diatomaceous earth | 42 parts by weight |

The above-mentioned components were pulverized and mixed to prepare a wettable powder, which would be applied after dilution with water.

Example 7

Granule

| | |
|---|---|
| Compound (I - 3) | 5 parts by weight |
| Bentonite | 43 parts by weight |
| Clay | 45 parts by weight |
| Lignin sulfonate | 7 parts by weight |

The above-mentioned components were uniformly mixed together with water and the mixture was granulated by an extrusion granulator, followed by drying.

Example 8

Emulsifiable concentrate

| | |
|---|---|
| Compound (I - 5) | 20 parts by weight |
| Polyoxyethylene alkylaryl ether | 10 parts by weight |
| Polyoxyethylene sorbitan monolaurate | 3 parts by weight |
| Xylene | 67 parts by weight |

The above-mentioned components were uniformly mixed together to prepare a composition of the form of emulsifiable concentrate.

Example 9

Test for controlling effect against Puccinia recondita on wheat

Onto the young seedlings of wheat of the second leaf stage (variety: NORIN No. 64, 16 seedlings per pot), which had been cultured while using unglazed pots of 10 cm in diameter, the wettable powder prepared in Example 6 was sprayed so as to be in a predetermined amount. After natural drying of the thus sprayed leave, an aqueous suspension of uredospores of Puccinia recondita collected from the attacked leaves of wheat was sprayed to inoculate on the thus dryed leaves, and the treated seedlings were kept for

24 hours at a temperature of 20 - 23°C in a high humidity atmosphere. Thereafter, the thus treated seedlings were left in a glass green house. After 7 - 10 days of the inoculation, the area rate of disease spots affected by Puccinia recondita was examined and the control value was calculated by the following formula.

$$\text{Control value} = \left(1 - \frac{\text{area rate of disease spots on treated pots}}{\text{area rate of disease spots on untreated pots}}\right) \times 100$$

The results are shown in Table 2.

Table 2

| Compound number (as in Table 1) | Concentration of spray (ppm) | Control value (%) |
|---|---|---|
| I - 1 | 3 | 100 |
| I - 2 | 3 | 100 |
| I - 3 | 3 | 99.5 |
| I - 4 | 3 | 86.3 |
| Racemic modification of I - 1 | 3 | 77.4 |
| Racemic modification of I - 2 | 3 | 80.2 |
| Racemic modification of I - 3 | 3 | 80.4 |
| Racemic modification of I - 4 | 3 | 55.2 |

Example 10

Test for controlling effect against Erysphe graminis f. sp. tritici on wheat

Onto the young seedlings of wheat of the first leaf stage (variety: NORIN No. 64, 16 seedlings per pot), which had been cultured while using unglazed pots of 10 cm in diameter, the granule prepared in Example 7 was scattered so as to be in a predetermined amount. After cultured in a green house for 7 days, an aqueous suspension of spores of Erysphe graminis f. sp. tritici collected from the attacked leaves of wheat was sprayed to inoculate on the leaves, and the treated seedlings were kept for 24 hours at a temperature of 20 - 24°C in a high humidity atmosphere. After 9 - 11 days of the inoculation, the area rate of disease spots affected by Erysphe graminis f. sp. tritici was examined and the control value was calculated by the following formula.

$$\text{Control value} = \left(1 - \frac{\text{area rate of disease spots on treated pots}}{\text{area rate of disease spots on untreated pots}}\right) \times 100$$

The results are shown in Table 3.

Table 3

| Compound number (as in Table 1) | Rate of scattering (kg/ha) | Control value (%) |
|---|---|---|
| I - 1 | 3 | 97.6 |
| I - 2 | 3 | 100 |
| I - 3 | 3 | 88.2 |
| I - 4 | 3 | 75.0 |
| Racemic modification of I - 1 | 3 | 58.8 |
| Racemic modification of I - 2 | 3 | 64.7 |
| Racemic modification of I - 3 | 3 | 60.0 |
| Racemic modification of I - 4 | 3 | 50.0 |

Example 11

Test for controlling effect against Botrytis cinerea on cucumber

Onto the cucumber plant of the first leaf stage (variety: SAGAMI HANPAKU), which had been cultured while using unglazed pots of 10 cm in diameter, a wettable powder prepared in Example 6 which was diluted in a predetermined concentration with water was sprayed at a rate of 5 ml per pot. After natural drying of the thus sprayed leave, a circular cutting of agar of a diameter of 4 mm containing Botrytis cinerea, which had been preliminarily cultured for 3 days at 20 °C while using a agar medium containing potato sucrose, was directly adhered to the center part of the leaf, and then the plant was kept at a temperature of 20 - 22°C in a high humidity atmosphere. After 3 days of the inoculation, the area rate of disease spots affected by Botrytis cinerea was examined and the control value was calculated by the following formula.

$$\text{Control value} = \left(1 - \frac{\text{area rate of disease spots on treated pots}}{\text{area rate of disease spots on untreated pots}}\right) \times 100$$

The results are shown in Table 4.

Table 4

| Compound number (as in Table 1) | Concentration of spray (ppm) | Control value (%) |
|---|---|---|
| I - 1 | 50 | 88.9 |
| I - 2 | 50 | 93.7 |
| I - 3 | 50 | 89.6 |
| I - 4 | 50 | 69.3 |
| Racemic modification of I - 1 | 50 | 73.6 |
| Racemic modification of I - 2 | 50 | 75.2 |
| Racemic modification of I - 3 | 50 | 71.7 |
| Racemic modification of I - 4 | 50 | 43.7 |

Example 12

Test for controlling effect against Pyricularia oryzae on rice plant

Onto the young seedlings of rice plant of the fourth leaf stage (variety: KOSHIHIKARI, 16 seedlings per pot), which had been cultured while using unglazed pots of 10 cm in diameter, a wettable powder prepared in Example 6 which was diluted in a predetermined concentration with water was sprayed at a rate of 5 ml per pot. After natural drying of the thus sprayed leaves, an aqueous suspension of spores of Pyricularia oryzae preriminarily cultured on the cultur medium was sprayed to inoculate on the thus dryed leaves. The treated seedlings were kept for 48 hours at a temperature of 26 - 28° C in a high humidity atmosphere and they were then left in a green house at 25 - 27 °C. After 6 - 8 days of the inoculation, the area rate of disease spots affected by Pyricularia oryzae was examined and the control value was calculated by the following formula.

$$\text{Control value} = \left(1 - \frac{\text{area rate of disease spots on treated pots}}{\text{area rate of disease spots on untreated pots}}\right) \times 100$$

The results are shown in Table 5.

Table 5

| Compound number (as in Table 1) | Concentration of spray (ppm) | Control value (%) |
|---|---|---|
| I - 1 | 50 | 83.5 |
| I - 2 | 50 | 85.2 |
| I - 3 | 50 | 81.9 |
| I - 4 | 50 | 79.7 |
| Racemic modification of I - 1 | 50 | 60.1 |
| Racemic modification of I - 2 | 50 | 64.9 |
| Racemic modification of I - 3 | 50 | 62.5 |
| Racemic modification of I - 4 | 50 | 45.8 |

Example 13

Test for controlling effect against Cochliobolus miyabeanus on rice plant

Onto the young seedlings of rice plant of the fifth leaf stage (variety: KOSHIHIKARI, 16 seedlings per pot), which had been cultured while using unglazed pots of 10 cm in diameter, a wettable powder prepared in Example 6 which was diluted in a predetermined concentration with water was sprayed at a rate of 5 ml per pot. After natural drying of the thus sprayed leaves, an aqueous suspension of spores of Cochliobolus miyabeanus preriminarily cultured on the cultur medium was sprayed to inoculate on the thus dryed leaves. The treated seedlings were kept for 48 hours at a temperature of 26 - 28 °C in a high humidity atmosphere and they were then left in a green house at 25 - 27 °C. After 6 - 8 days of the inoculation, the area rate of disease spots affected by Cochliobolus miyabeanus was examined and the control value was calculated by the following formula.

$$\text{Control value} = \left(1 - \frac{\text{area rate of disease spots on treated pots}}{\text{area rate of disease spots on untreated pots}}\right) \times 100$$

The results are shown in Table 6.

Table 6

| Compound number (as in Table 1) | Concentration of spray (ppm) | Control value (%) |
|---|---|---|
| I - 1 | 25 | 89.1 |
| I - 2 | 25 | 95.5 |
| I - 3 | 25 | 91.3 |
| I - 4 | 25 | 82.6 |
| Racemic modification of I - 1 | 25 | 75.9 |
| Racemic modification of I - 2 | 25 | 80.4 |
| Racemic modification of I - 3 | 25 | 67.5 |
| Racemic modification of I - 4 | 25 | 50.8 |

Example 14

Test of plant growth controlling effect

Onto the young seedlings of rice plant in the begining of green leaf stage (variety: SASANISHIKI) grown in planters (30 X 60 X 3 cm), an emulsion prepared in Example 8 which was diluted in a predetermined concentration with water was uniformly poured at a rate of 500 ml per planter. After cultured for 14 days in a green house, the plant length was measured and length controlling rate was calculated by the following formula

$$\text{Length controlling rate (\%)} = \left(1 - \frac{\text{Length in treated area}}{\text{Length in untreated area}}\right) \times 100$$

The results are shown in Table 7.

Table 7

| Compound number (as in Table 1) | Concentration of emulsion (ppm) | Length controlling rate (%) |
|---|---|---|
| I - 1 | 500 | 0 |
| I - 2 | 500 | 0 |
| I - 3 | 500 | 0 |
| I - 4 | 500 | 0 |
| Racemic modification of I - 1 | 500 | 32.1 |
| Racemic modification of I - 2 | 500 | 40.8 |
| Racemic modification of I - 3 | 500 | 28.2 |
| Racemic modification of I - 4 | 500 | 17.8 |

## Claims

1. An optically active (-)-triazole derivative represented by the following general formula (I), which has a configuration that $R^1$ and the nonsubstituted or substituted phenylmethyl group are bonded to the cis-position of the hydroxyl group and $R^2$ is bondend to the trans-position of the hydroxyl group:

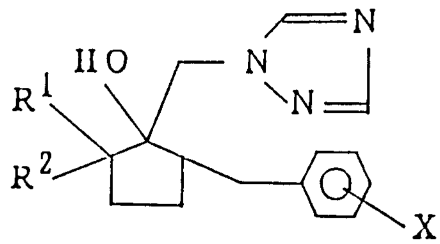

(I)

wherein one of $R^1$ and $R^2$ is a $C_2$ - $C_4$ straight-chain alkyl group and the other is a hydrogen atom, and X denotes a hydrogen atom or a halogen atom.

2. An optically active (-)-triazole derivative of Claim 1, wherein $R^1$ = $C_2H_5$, $R^2$ = H and X = 4-Cl.

3. An optically active (-)-triazole derivative of Claim 1, wherein $R^1$ = H, $R^2$ = $C_2H_5$ and X = 4-Cl.

4. An optically active (-)-triazole derivative of Claim 1, wherein $R^1$ = n-$C_3H_7$, $R^2$ = H and X = 4-Cl.

5. An optically active (-)-triazole derivative of Claim 1, wherein $R^1$ = n-$C_4H_9$, $R^2$ = H and X = 4-Cl.

6. An agricultural and horticultrual fungicide composition comprising as an active ingredient an optically active (-)-triazole derivative represented by the following general formula (I), which has a configuration that $R^1$ and the nonsubstituted or substituted phenylmethyl group are bonded to the cis-position of the hydroxyl group and $R^2$ is bondend to the trans-position of the hydroxyl group,

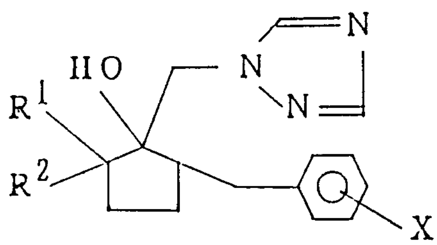

(I)

wherein one of $R^1$ and $R^2$ is a $C_2$ - $C_4$ straight-chain alkyl group and the other is a hydrogen atom, and X denotes a hydrogen atom or a halogen atom;
together with a carrier or other adjuvants.

7. An agricultural and horticultrual fungicide composition of Claim 6, wherein $R^1$ = $C_2H_5$, $R^2$ = H and X = 4-Cl.

8. An agricultural and horticultrual fungicide composition of Claim 6, wherein $R^1$ = H, $R^2$ = $C_2H_5$ and X = 4-Cl.

9. An agricultural and horticultrual fungicide composition of Claim 6, wherein $R^1$ = n-$C_3H_7$, $R^2$ = H and X = 4-Cl.

10. An agricultural and horticultrual fungicide composition of Claim 6, wherein $R^1$ = n-$C_4H_9$, $R^2$ = H and X = 4-Cl.

European Patent Office

# EUROPEAN SEARCH REPORT

Application Number

EP 91 12 0483

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,D<br><br><br><br>D | EP-A-0 267 778 (KUREHA KAGAKU KOGYO KK)<br>*page 35, line 59 - page 36, line 4; table 1, compounds no. 29, 30, 37, 38, 42, 44, 67, 71; claims 1, 6*<br>& JP-A-01 093 574 (...)<br>----- | 1,6 | C07D249/08<br>A01N43/653 |
| | | | **TECHNICAL FIELDS SEARCHED (Int. Cl.5 )** |
| | | | C07D<br>A01N |

The present search report has been drawn up for all claims

| Place of search | Date of completion of the search | Examiner |
|---|---|---|
| BERLIN | 03 MARCH 1992 | van Amsterdam |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)